# EUROPÄISCHE PATENTSCHRIFT

(11) **EP 1 392 698 B1**
(45) Veröffentlichungstag und Bekanntmachung des Hinweises auf die Patenterteilung: **13.10.2004**
(21) Anmeldenummer: 02718203.9
(22) Anmeldetag: 26.03.2002
(51) Int. Cl.: C07D 495/16

(54) **VERFAHREN ZUR HERSTELLUNG VON 6-ARYL-4H-S-TRIAZOLO[3,4-C]-THIENO[2,3-E]-1,4-DIAZEPINEN**
METHOD FOR THE PRODUCTION OF 6-ARYL-4H-S-TRIAZOLO[3,4-C]-THIENO[2,3-E]-1,4-DIAZEPINES
PROCEDE DE FABRICATION DE 6-ARYL-4H-S-TRIAZOLO[3,4-C]-THIENO[2,3-E]-1,4-DIAZEPINES

(30) Priorität: 04.04.2001 DE 10116378
(43) Veröffentlichungstag der Anmeldung: 03.03.2004
(73) Patentinhaber: Boehringer Ingelheim Pharma GmbH & Co.KG, 55216 Ingelheim am Rhein (DE)
(72) Erfinder: LOCK, Ralf, 55122 Mainz (DE); BELZER, Werner, 56329 St. Goar (DE)
(86) Internationale Anmeldenummer: PCT/EP2002/003352
(87) Internationale Veröffentlichungsnummer: WO 2002/081479

(56) Entgegenhaltungen:
- US-A- 4 094 984
- KAWAKAMI Y ET AL: "Structural optimization of 4-(2-chlorophenyl)-9-methyl-6H-thieno[3,2- f]-[1,2,4]triazolo[4,3-a][1,4]diaz epines as antagonists for platelet activating factor: pharmacological contribution of substituents at the 2- and 6-positions of a condensed ring system" EUROPEAN JOURNAL OF MEDICINAL CHEMISTRY, EDITIONS SCIENTIFIQUE ELSEVIER, PARIS, FR, Bd. 31, Nr. 9, 1996, Seiten 683-692, XP004040246 ISSN: 0223-5234

## Beschreibung

Die Erfindung betrifft ein Verfahren zur Herstellung 6-Aryl-4H-s-triazolo[3,4-c]-thieno[2,3-e]-1,4-diazepinen der Formel I, 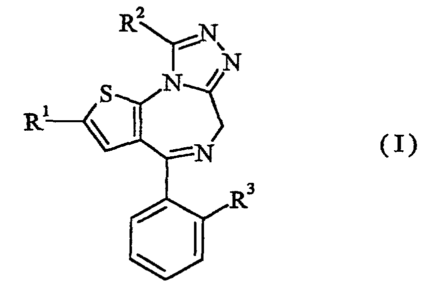 worin R¹, R² und R³ die angegebene Bedeutung besitzen, aus einem 5-Aryl-1,3-dihydrothieno[2,3-*e*]-1,4-diazepin-2-on der Formel II

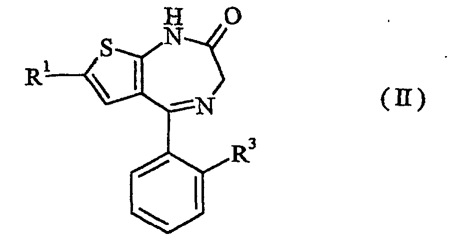

### Hintergrund der Erfindung

6-Aryl-4H-s-triazolo[3,4-*c*]-thieno[2,3-*e*]-1,4-diazepine, insbesondere Brotizolam (R¹ = Br, R² = Methyl, R³ = Cl) sind zum Beispiel bekannt aus dem US Patent US 4,094,984 und stellen wertvolle Arzneimittel dar, die insbesondere sedative Eigenschaften aufweisen und bei Schlafstörungen eingesetzt werden können.

Die dort beschriebene Herstellung ausgehend von einem 5-Aryl-1,3-dihydrothieno[2,3-*e*]-1,4-diazepin-2-on der Formel II ist jedoch für eine Produktion im technischen Maßstab wenig geeignet, da die dabei durchlaufenen Zwischenprodukte, zum Beispiel 5-Aryl-1,3-dihydrothieno[2,3-*e*]-1,4-diazepin-2-thione isoliert werden müssen, und bei deren Umsetzung übelriechende und teilweise giftige Thiole freigesetzt werden, die zu einer Kontamination der Produkte führen können.

Y. Kawakami et. al., Eur. J. Med. Chem. (1996) 31, 683-692 offenbart Aryl-1,4-diazypin-Derivate und ihne Herstellung ausgehend von 2-Amino-N-[3-(2-chlorobenzoyl)-5-hexyl-thiophen-2-yl]-acetamid.

Es ist daher Aufgabe der vorliegenden Erfindung, ein Verfahren bereitzustellen, das die großtechnisch durchführbare Synthese, Aufarbeitung, Reinigung und Isolierung von 6-Aryl-4H-s-triazolo[3,4-*c*]-thieno[2,3-*e*]-1,4-diazepinen der Formel I erlaubt, bei der die vorstehend genannten Nachteile überwunden werden.

### Detaillierte Beschreibung der Erfindung

Überraschenderweise wurde gefunden, dass 6-Aryl-4H-s-triazola[3,4-*c*]-thiena[2,3-*e*]-1,4-diazepine der Formel I, worin
R¹ für ein Wasserstoff- oder Halogenatom oder einen C₁-C₆ Alkylrest steht,
R² für ein Wasserstoff- oder Halogenatom oder einen C₁-C₆ Alkyl-, C₁-C₆ Hydroxyalkyl-, C₃-C₆ Cycloalkylrest oder eine 5- oder 6-gliedrigen Sauerstoff-, Schwefel- oder Stickstoffhaltige Heterocyclische Gruppe steht, die gegebenenfalls am Stickstoffatom durch einen C₁-C₃ Alkylrest substituiert sein kann, und
R³ für ein Wasserstoff- oder Halogenatom steht,
in großtechnischem Maßstab hergestellt werden können, indem man ein 5-Aryl-1,3-dihydrothieno[2,3-*e*]-1,4-diazepin-2-on der Formel II,
worin R¹ und R³ die angegebene Bedeutung aufweisen,
(a) mit einem Chlorierungsmittel behandelt,
(b) das erhaltene 5-Aryl-2-chlorthieno[2,3-*e*]-1,4-diazepin der Formel III, 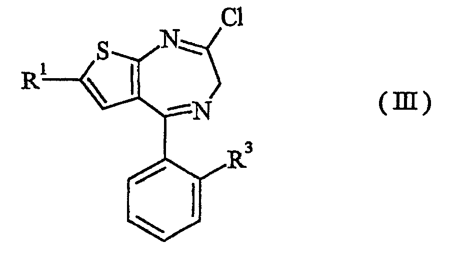 worin R¹ und R³ die angegebene Bedeutung aufweisen, mit einem Acylhydrazin der Formel IV

   R²-CO-NH-NH₂ (IV)

   worin R² die angegebene Bedeutung aufweist, umsetzt, und
(c) das so erhaltene Produkt mit einer Base behandelt.

Gegenstand der Erfindung ist somit ein Verfahren zur Herstellung der Verbindungen der Formel I aus Verbindungen der Formel II gemäß der oben angeführten Schritte (a), (b) und (c).

Ein weiterer Gegenstand der Erfindung ist ein Verfahren zur Herstellung einer Verbindung Formel I, worin R¹ für Brom steht, wobei man eine Verbindung der Formel V, 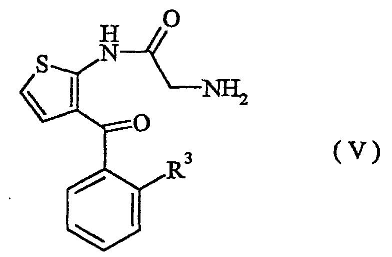 worin R³ die angegebene Bedeutung besitzt,
nacheinander folgenden Reaktionen unterwirft:
(d) Cyclisierung unter Wasser-abspaltenden Bedingungen;
(e) Umsetzung des in Schritt d) erhaltenen Produkts mit einem Bromierungsmittel;
(f) Überführung der so erhaltenen Verbindung der Formel II, worin R¹ für Brom steht, in die Verbindung der Formel I gemäß der Schritte (a) bis (c),
dadurch gekennzeichnet, dass man die Schritte (d) und (e) in einem Eintopfverfahren durchführt.

Weiterhin Gegenstand der Erfindung sind die 5-Aryl-2-chlorothieno[2,3-e]-1,4-diazepineder Formel III, 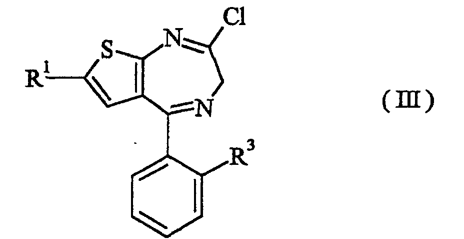 worin R¹ und R³ die Bedeutungen aufweisen, insbesondere 7-Brom-5-(2-chlorphenyl)-2-chlorothieno[2,3-*e*]-1,4-diazepin.

Der Begriff "Alkyl" wie er vor- und nachstehend bezüglich der Gruppen R¹ und/oder R² verwendet wird, steht für eine geradkettige oder verzweigte Alkylgruppe mit bis zu 6 C-Atomen, vorzugsweise 1 bis 4 C-Atomen. Besonders bevorzugt sind Methyl, Ethyl, *n*-Propyl, *i*-Propyl, *n*-Butyl und *tert-*Butyl, insbesondere Methyl.

Der Begriff "Hydroxyalkyl" wie er vor- und nachstehend bezüglich der Gruppe R² verwendet wird, steht für eine geradkettige oder verzweigte Alkylgruppe mit bis zu 6 C-Atomen, vorzugsweise 1 bis 4 C-Atomen, welche durch eine Hydroxygruppe substituiert. Bevorzugt sind ω-Hydroxyalkylgruppen mit 1 bis 3 C-Atomen, insbesondere Hydroxymethyl, 2-Hydroxyethyl und 3-Hydroxypropyl.

Der Begriff "Cycloalkyl" wie er vor- und nachstehend bezüglich der Gruppe R² verwendet wird, steht für eine cyclische Alkylgruppe mit 3 bis 6 C-Atomen, vorzugsweise 5 oder 6 C-Atomen, insbesondere Cyclopentyl und Cyclohexyl.

Der Begriff "Heterocyclische Gruppe" wie er vor- und nachstehend bezüglich der Gruppe R² verwendet wird, steht für einen gesättigten oder ungesättigten 5- oder 6-gliedrigen, Heterocyclylrest, welcher neben Kohlenstoffatomen und mindestens ein Heteroatom ausgewählt aus der Gruppe Stickstoff Sauerstoff und Schwefel aufweist. Dabei sind folgende Heterocyclylgruppen bevorzugt:
gesättigte oder aromatische 5- oder 6-gliedrige Heterocyclylgruppen, die
- die ein oder zwei Stickstoffatome aufweisen, insbesondere Pyrrolidyl, Pyrazolyl, Imidazolyl, Pyridyl, Pyrimidyl, Piperidyl und Piperazyl, oder
- ein Stickstoffatom und ein Sauerstoff- oder Schwefelatom aufweisen, insbesondere. Morpholino und Thiomorpholino,
- ein Sauerstoffatom aufweisen, insbesondere Tetrahydropyranyl und Tetrahydrofuranyl.

Der Begriff "Eintopf-Verfahren" wie er vor- oder nachstehend für die Abfolge bestimmter Reaktionsschritte verwendet wird, steht für die Abfolge von zwei oder mehreren aufeinander folgenden Syntheseschritten, wobei das in einem ersten oder zweiten Syntheseschritt erhaltene Zwischenprodukt ohne Isolation bzw. Reinigung im folgenden Syntheseschritt weiterverwendet wird. Vorzugsweise erfolgt die nachfolgende Reaktionsstufe im gleichen Reaktionsgefäß und gegebenenfalls in Gegenwart des Reaktionsmediums, in welchem der vorangehende Reaktionsschritt durchgeführt wurde.

In einer bevorzugten Ausführungsform weisen die Reste R¹ bis R³ folgende Bedeutungen auf:
R¹ Halogen oder Methyl, insbesondere Brom,
R² Wasserstoff oder Methyl, insbesondere Methyl, und
R³ Halogen, insbesondere Chlor.

In einer bevorzugten Ausführungsform des erfindungsgemäßen Verfahrens zur Herstellung der Verbindung der Formel I wird:
- in Schritt (a) die Verbindung der Formel (II) mit Phosphorpentachlorid in Gegenwart eines inerten Verdünnungsmittels und einem tertiären Amin umgesetzt;
- in Schritt (b) die Verbindung der Formel (III) bei einer Temperatur unterhalb von 100 °C mit Essigsäurehydrazid in Gegenwart eines inerten Verdünnungsmittels umgesetzt.
- in Schritt (c) die in Schritt (b) erhaltene Verbindung bei einer Temperatur von 0 °C bis 50 °C mit wässriger Natronlauge behandelt;
- die Reaktionsfolge der Schritte (a) bis (c) in einem Eintopf Verfahren durchgeführt.

### Stufe II → III

### Schritt (a)

Die Umsetzung der Verbindung der Formel II mit dem Chlorierungsmittel erfolgt in der Regel in Gegenwart eines inerten Verdünnungsmittels.

Vorzugsweise wird die Verbindung der Formel II mit einem Chlorierungsmittel ausgewählt aus der Gruppe Thionylchlorid, Sulfurylchlorid, Titantetrachlorid, Oxalylchlorid, Phosgen, Di- und Triphosgen, Phosphortrichlorid, Phosphoroxychlorid, Phosphorpentachlorid und Antimonpentachlorid umgesetzt, zweckmäßigerweise in einem Verdünnungsmittel wie Tetrahydrofuran, Dioxan, Toluol, Dichlormethan, Chloroform oder Chlorbenzol, oder Gemischen dieser Verdünnungsmittel, gegebenenfalls in Gegenwart einer organischen Base, z.B. Triethylamin, *N*-Ethyldiisopropylamin, Pyridin oder 4-Dimethylaminopyridin, bei Temperaturen von - 20 bis + 60 °C, vorzugsweise -10 bis + 20 °C. Die Reaktion wird in der Regel bei Normaldruck durchgeführt, wobei 0,9 bis 2,0, vorzugsweise 1,1 bis 1,9, insbesondere 1,2 bis 1,4 Mol eines Chlorierungsmittels bezogen auf 1 Mol der Verbindung der Formel II eingesetzt werden. Die Umsetzung ist in der Regel unter den angegebenen Bedingungen nach 15 bis 600 Minuten, vorzugsweise 30 bis 180 Minuten beendet. Das erhaltene Rohprodukt wird in der Regel ohne weitere Aufreinigung im nächsten Schritt weiterverarbeitet.

In einer besonders bevorzugten Ausführungsform des erfindungsgemäßen Verfahrens wird zu einer Suspension von 1,1 bis 1,5 Mol Phosphorpentachlorid bezogen auf 1 Mol der Verbindung der Formel II in Dichlormethan bei 0 bis 20 °C unter Kühlung eine Suspension der Verbindung der Formel II in Pyridin und Dichlormethan getropft. Man lässt 30 bis 120 Minuten bei etwa 10 °C nachrühren.

### Stufe III → I

### Schritt (b)

Die Umsetzung der Verbindung der Formel III mit der Verbindung der Formel IV erfolgt in der Regel in einem inerten Lösungsmittel.

Vorzugsweise wird die Verbindung der Formel III mit einer Verbindung der Formel IV, insbesondere Essigsäurehydrazid umgesetzt, zweckmäßigerweise in einem Verdünnungsmittel wie Tetrahydrofuran, Dioxan, Toluol, Dichlormethan, Chloroform oder Chlorbenzol, oder Gemischen dieser Verdünnungsmittel, insbesondere in einem Gemisch aus Dichlormethan und Tetrahydrofuran, gegebenenfalls in Gegenwart einer organischen Base, z.B. Triethylamin, *N*-Ethyldiisopropylamin, Pyridin oder 4-Dimethylaminopyridin und der in Schritt (a) entstandenen weiteren Reaktionsprodukte, z.B. Phosphoroxychlorid, bei Temperaturen von 0 bis 100 °C, vorzugsweise 10 bis + 90 °C. Die Reaktion wird in der Regel bei Normaldruck durchgeführt, wobei 0,9 bis 3,0, vorzugsweise 1,5 bis 2,5, insbesondere 1,8 bis 2,2 Mol einer Verbindung der Formel IV bezogen auf 1 Mol der Verbindung der Formel III eingesetzt werden. Die Umsetzung ist in der Regel unter den angegebenen Bedingungen nach 1 bis 48 Stunden, vorzugsweise 5 bis 24 Stunden beendet. Das erhaltene Rohprodukt wird in der Regel ohne weitere Aufreinigung im nächsten Schritt weiterverarbeitet.

In einer besonders bevorzugten Ausführungsform des erfindungsgemäßen Verfahrens tropft man unter Kühlung bei 0-30 °C eine erwärmte Lösung von etwa 2 Äquivalenten Essigsäurehydrazid in Tetrahydrofuran bezogen auf 1 Äquivalent der Formel III zu der in Schritt (a) erhaltenen Reaktionsmischung. Anschließend rührt man 0,5 bis 2 Stunden bei Raumtemperatur nach und erwärmt dann 5-20 Stunden unter Rückfluss. Dabei entsteht bereits das Brotizolam als Hydrochlorid/Phosphat, mit einer anschließend zugesetzten Base wird das Brotizolam freigesetzt.

### Schritt (c)

Die Umsetzung der in Schritt (b) erhaltenen Verbindung mit der Base erfolgt in der Regel in Gegenwart eines inerten Lösungsmittels.

Vorzugsweise wird die in Schritt (b) erhaltene Verbindung mit einer wässrigen Base, insbesondere Natriumhydroxid umgesetzt, zweckmäßigerweise in einem Verdünnungsmittel wie Wasser, Methanol, Ethanol, Tetrahydrofuran, Dioxan, Toluol, Dichlormethan, Chloroform, Aceton, Methylethylketon oder Chlorbenzol, oder Gemischen dieser Verdünnungsmittel, insbesondere in einem Gemisch aus Dichlormethan, Tetrahydrofuran, Wasser, gegebenenfalls in Gegenwart einer organischen Base, z.B. Triethylamin, *N*-Ethyldiisopropylamin, Pyridin oder 4-Dimethylaminopyridin und der in den Schritten (a) und (b) entstandenen weiteren Reaktionsprodukte, z.B. Phosphoroxychlorid, bei Temperaturen von 0 bis 120 °C, vorzugsweise 10 bis 100 °C. Die Reaktion wird in der Regel bei Normaldruck durchgeführt, wobei 1,0 bis 10, vorzugsweise 6 bis 9 Mol einer Base bezogen auf 1 Mol des Chlorierungsreagenzes eingesetzt werden. Die Umsetzung ist in der Regel unter den angegebenen Bedingungen bereits nach wenigen Minuten bis Stunden, 0,1 bis 24 Stunden, vorzugsweise 0,1-2h beendet. Das erhaltene Rohprodukt wird in der Regel durch Extraktion und/oder Kristallisation isoliert und gegebenenfalls durch Umkristallisation gereinigt.

In einer besonders bevorzugten Ausführungsform des erfindungsgemäßen Verfahrens gibt man unter Kühlung Wasser zu der in Schritt (b) erhaltenen Reaktionsmischung und stellt mit Natronlauge einen alkalischen pH-Wert von 7,5-13, insbesondere 9-11 ein. Die Phasen werden getrennt und die wässrige Phase mit Dichlormethan extrahiert. Die vereinigten organischen Phasen werden vorzugsweise mit Aktivkohle entfärbt, filtriert und im Vakuum zur Trockene eingeengt. Der Rückstand wird in siedendem Methylethylketon aufgenommen, erneut entfärbt, filtriert, und anschließend eingeengt. Man kühlt auf 0 bis 10 °C ab und rührt 2 Stunden bei dieser Temperatur nach. Das auskristallisierte Produkt wird abfiltriert, mit Methylethylketon und Essigsäurethylester gewaschen und getrocknet.

In einer bevorzugten Ausfdhrungsform des erfindungsgemäßen Verfahrens zur Herstellung der Verbindung der Formel II wird:
- die Verbindung der Formel V in Schritt (d) mit einer Säure in einem hochsiedendem Lösungsmittel erhitzt;
- die in Schritt (d) erhaltene Verbindung mit Brom in Gegenwart eines tertiären Amins umsetzt.

### Stufe V → II

### Schritt (d)

Die Cyclisierung der Verbindung der Formel V erfolgt in der Regel in Gegenwart eines inerten Verdünnungsmittels.

Vorzugsweise wird die Verbindung der Formel V mit einer Säure ausgewählt aus der Gruppe Salzsäure, Schwefelsäure, p-Toluolsulfonsäure Phosphorsäure, einer Carbonsäure wie Essigsäure, Propionsäure, Pivalinsäure, Trifluoressigsäure und Kieselgel gegebenenfalls in Gegenwart einer Base wie Pyridin, zweckmäßigerweise in einem hochsiedenden Verdünnungsmittel wie *n*-Butanol, Amylalkohol, Toluol, Xylol, Chlorbenzol, oder Gemischen dieser Verdünnungsmittel bei Temperaturen von 20 bis 160 °C, vorzugsweise 60 bis 140°C umgesetzt. Die Reaktion wird in der Regel bei Normaldruck durchgeführt, wobei 1,5 bis 6,0, vorzugsweise 1,9 bis 4,0, insbesondere 3,2 bis3,8 Mol einer Säure bezogen auf 1 Mol der Verbindung der Formel V eingesetzt werden. Die Umsetzung ist in der Regel unter den angegebenen Bedingungen nach 15 bis 600 Minuten, vorzugsweise 30 bis 180 Minuten beendet. Das erhaltene Rohprodukt wird in der Regel ohne weitere Aufreinigung im nächsten Schritt weiterverarbeitet.

In einer besonders bevorzugten Ausführungsform des erfindungsgemäßen Verfahrens wird in eine Mischung aus 4 bis 12 Teilen n-Butanol und 0,4 bis 1,2 Teilen Eisessig bei 80-110 °C ein Teil von 2-Amino-*N*-[3-(2-chlorbenzoyl)thiophen-2-yl]acetamid eingetragen und 0,5 bis 2 Stunden unter Rückfluss erhitzt.

### Schritt (e)

Die Umsetzung der in Schritt (d) erhaltenen Verbindung mit einem Bromierungsmittel erfolgt in der Regel in Gegenwart eines inerten Lösungsmittels.

Vorzugsweise wird die in Schritt (d) erhaltene Verbindung mit einem Bromierungsmittel ausgewählt aus der Gruppe Brom, N-Bromsuccinimid und 1,3-Dibrom-5,5-dimethylhydantoin umgesetzt, zweckmäßigerweise in einem Verdünnungsmittel wie n-Butanol, Amylalkohol, Toluol, Xylol, Chlorbenzol, oder Gemischen dieser Verdünnungsmittel gegebenenfalls in Gegenwart einer organischen Base, z.B. Triethylamin, *N*-Ethyldiisopropylamin oder Pyridin, bei Temperaturen von - 20 bis + 60 °C, vorzugsweise -10 bis + 20 °C. Die Reaktion wird in der Regel bei Normaldruck durchgeführt, wobei 0,9 bis 2,0, vorzugsweise 1,1 bis 1,9, insbesondere 1,2 bis 1,8 Mol eines Bromierungsmittels bezogen auf 1 Mol der Verbindung der Formel V eingesetzt werden. Die Umsetzung ist in der Regel unter den angegebenen Bedingungen nach 15 bis 600 Minuten, vorzugsweise 30 bis 240 Minuten beendet Das erhaltene Rohprodukt wird in der Regel durch Kristallisation isoliert und gegebenenfalls durch Umkristallisation gereinigt.

In einer besonders bevorzugten Ausiuhrungsform des erfindungsgemäßen Verfahrens kühlt man die in Schritt (d) erhaltene Reaktionsmischung auf Raumtemperatur und gibt Pyridin und etwa 1,2 bis 1,8 Mol Brom bezogen auf 1 Mol der eingesetzten Verbindung der Formel V zu. Anschließend lässt man 0,1 bis 4 Stunden nachrühren, kühlt auf 0-20 °C und rührt weitere 1 bis 3 Stunden bei dieser Temperatur. Das auskristallisierte Produkt wird durch Filtration isoliert, mit wässrigem Ethanol gewaschen und getrocknet

Die als Ausgangsmaterial benötigte Verbindung der Formel V ist zum Beispiel aus der deutschen Offenlegungsschrift DE 22 17 157 oder dem US Patent US 4,094,984 bekannt und kann in an sich bekannter Weise durch Umsetzung eines 2-Amino-3-arylthiophens mit Bromacetylbromid und Ammoniak hergestellt werden.

Weitere vorteilhafte Aspekte der erfindungsgemäßen Vorgehensweise sind die hohe Raum-Zeit-Ausbeute beim vorliegenden Prozess sowie die hohe Ausbeute und Reinheit an den jeweiligen Zwischenprodukten, welche ohne chromatographische Aufreinigung weiterverarbeitet werden können.

Die nachfolgenden Beispiele dienen der Illustration exemplarisch durchgeführter Verfahren zur Herstellung der Verbindung der Formel I. Sie sind lediglich als mögliche, exemplarisch dargestellte Vorgehensweisen zu verstehen, ohne die Erfindung auf deren Inhalt zu beschränken.

### Beispiel 1

### 7-Brom-5-(2-chlorphenyl)-1,3-dihydrothieno[2,3-e]-1,4-diazepin-2-on, (1)

In eine Mischung aus 1,81 n-Butanol und 150 ml Eisessig werden bei 110°C 220 g (0,746 mol) 2-Amino-*N*-[3-(2-chlorbenzoyl)thiophen-2-yl]acetamid, (hergestellt durch Umsetzen von 2-Amino-3-(2-chlorbenzoyl)thiophen mit Bromacetylbromid und anschließender Einleitung von gasförmigen Ammoniak) eingetragen. Man erhitzt 1 Stunde unter Rückfluss, kühlt auf Raumtemperatur und gibt unter Kühlung 330 ml Pyridin und 57 ml (178,8 g, 1,12 mol) Brom zu. Anschließend lässt man 0,5 bis 2 Stunden nachrühren, kühlt auf 5 bis 10 °C und rührt weitere 2 Stunden bei dieser Temperatur. Das auskristallisierte Produkt wird durch Filtration isoliert, mit 50 %-igem Ethanol gewaschen und über Nacht im Vakuum bei 60 °C getrocknet. Ausbeute: 152 g (57.3 %).

### Beispiel 2

### Brotizolam

In eine Suspension von 36 g (0,183 mol) Phosphorpentachlorid in 250 ml Dichlormethan wird bei 5-10 °C unter Kühlung eine Suspension von 50 g (0,14 mol) (1) in 2,5 ml Pyridin und 200 ml Dichlormethan getropft. Man lässt 1 Stunde bei 10 °C nachrühren und tropft unter Kühlung bei 10-20 °C eine auf 40-50 °C erwärmte Lösung von 17 g (0,28 mol) Essigsäurehydrazid in 170 ml Tetrahydrofuran zu. Anschließend rührt man 1 Stunde bei Raumtemperatur nach und erwärmt dann 5 bis 18 Stunden unter Rückfluss. Danach kühlt man auf Raumtemperatur ab, gibt unter Kühlung 380 ml Wasser zu und stellt mit 45 %iger Natronlauge alkalischen pH-Wert ein. Die Phasen werden getrennt und die wässrige Phase mit 100 ml Dichlormethan extrahiert. Die vereinigten organischen Phasen werden mit 5 g Aktivkohle versetzt, 10 Minuten gerührt, filtriert und im Vakuum zur Trockene eingeengt. Der Rückstand wird in 750ml siedendem Methylethylketon aufgenommen, mit 5g Aktivkohle versetzt und weitere 10min unter Rückfluss erhitzt. Die Suspension wird filtriert, anschließend werden 570 ml Lösungsmittel abdestilliert. Man lässt langsam auf Raumtemperatur erkalten und kühlt dann auf 5 °C ab und rührt 2 Stunden bei dieser Temperatur nach. Das auskristallisierte Produkt wird abfiltriert, mit 30 ml kaltem Methylethylketon sowie 50 ml kaltem Essigester gewaschen und über Nacht bei 60 °C im Vakuum getrocknet. Ausbeute: 36,6g (66 %) Brotizolam.

## Patentansprüche

1. Verfahren zur Herstellung von 6-Aryl-4H-s-triazolo[3,4-*c*]-thieno[2,3-*e*]-1,4-diazepinen der Formel I, 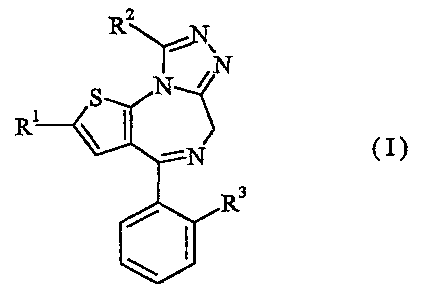 wobei
R¹ für ein Wasserstoff- oder Halogenatom oder einen C₁-C₆ Alkylrest steht,
R² für ein Wasserstoff- oder Halogenatom oder einen C₁-C₆ Alkyl-, C₁-C₆ Hydroxyalkyl-, C₃-C₆ Cycloalkylrest oder eine 5- oder 6-gliedrigen Sauerstoff-, Schwefel- oder Stickstoffhaltigen Heterocyclische Gruppe steht, die gegebenenfalls am Stickstoffatom durch einen C₁-C₃ Alkylrest substituiert sein kann, und
R³ für ein Wasserstoff oder Halogenatom steht,
aus einem 5-Aryl-1,3-dihydrothieno[2,3-*e*]-1,4-diazepin-2-on der Formel II 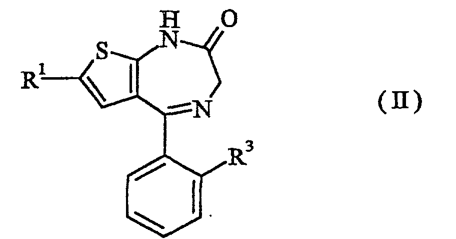 worin R¹ und R³ die angegebene Bedeutung aufweisen, **dadurch gekennzeichnet, dass** man die Verbindung der Formel II,
(a) mit einem Chlorierungsmittel behandelt,
(b) das erhaltene 5-Aryl-2-chlorthieno[2,3-*e*]-1,4-diazepin der Formel III, 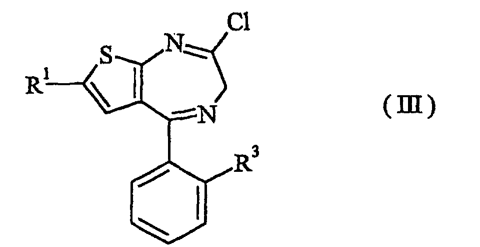 worin R¹ und R³ die angegebene Bedeutung aufweisen, mit einem Acylhydrazin der Formel IV
R²-CO-NH-NH₂ (IV)
worin R² die angegebene Bedeutung aufweist, umsetzt, und
(c) das so erhaltene Produkt mit einer Base behandelt.

2. Verfahren nach Anspruch 1zur Herstellung einer Verbindung der Formel I, worin R¹ für ein Bromatom steht, R² für eine Methylgruppe steht, und R³ für ein Chloratom steht.

3. Verfahren nach Anspruch 1 oder 2, **dadurch gekennzeichnet, dass** man die Schritte (a) bis (c) in einem Eintopf-Verfahren durchführt.

4. Verfahren nach einem der Ansprüche 1 bis 3, **dadurch gekennzeichnet, dass** man in Schritt (a) die Verbindung der Formel (II) mit Phosphorpentachlorid in Gegenwart eines inerten Verdünnungsmittels und eines tertiären Amins umsetzt.

5. Verfahren nach einem der Ansprüche 1 bis 4, **dadurch gekennzeichnet, dass** man in Schritt (b) die Verbindung der Formel (III) bei einer Temperatur unterhalb von 100 °C mit Essigsäurehydrazid in Gegenwart eines inerten Verdünnungsmittels umsetzt.

6. Verfahren nach einem der Ansprüche 1 bis 5, **dadurch gekennzeichnet, dass** man in Schritt (c) die in Schritt (b) erhaltene Verbindung bei einer Temperatur von 0 °C bis 50 °C mit wässriger Natronlauge behandelt.

7. Verfahren zur Herstellung einer Verbindung der Formel I, worin R¹ für Brom steht, wobei man eine Verbindung der Formel V, 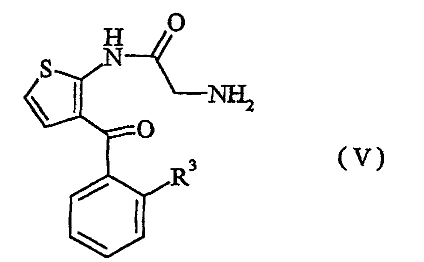 worin R³ die in Anspruch 1 oder 2 angegebene Bedeutung besitzt,
nacheinander folgenden Reaktionen unterwirft:
(d) Cyclisierung unter Wasser-abspaltenden Bedingungen;
(e) Umsetzung des in Schritt d) erhaltenen Produkts mit einem Bromierungsmittel;
(f) Überführung der so erhaltenen Verbindung der Formel II, worin R¹ für Brom steht, in die Verbindung der Formel I gemäß einem der Ansprüche 1 bis 6,
**dadurch gekennzeichnet, dass** man die Schritte (d) und (e) in einem Eintopf-Verfahren durchführt.

8. Verfahren nach Anspruch 7, **dadurch gekennzeichnet, dass** man die Verbindung der Formel V in Schritt (d) mit einer Säure in einem hochsiedenden Lösungsmittel erhitzt.

9. Verfahren nach Anspruch 7 oder 8, **dadurch gekennzeichnet, dass** man in Schritt (d) erhaltene Verbindung mit Brom in Gegenwart eines tertiären Amins umsetzt.

10. 5-Aryl-2-chlorothieno[2,3-*e*]-1,4-diazepin der Formel III, 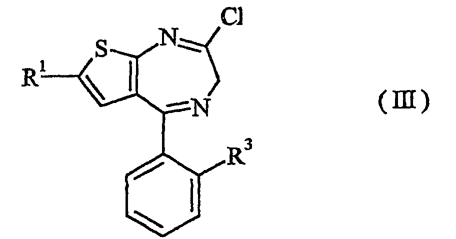 worin R¹ und R³ die in Anspruch 1 oder 2 angegebenen Bedeutungen aufweisen.

## Claims

1. Process for preparing 6-aryl-4H-s-triazolo[3,4-*c*]-thieno[2,3-*e*]-1,4-diazepines of formula I, 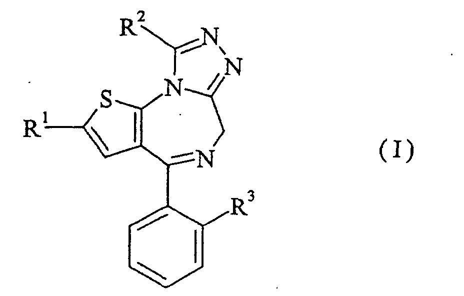 wherein
R¹ denotes a hydrogen or halogen atom or a C₁-C₆ alkyl group,
R² denotes a hydrogen or halogen atom or a C₁-C₆ alkyl, C₁-C₆ hydroxyalkyl, C₃-C₆ cycloalkyl group or a 5- or 6-membered oxygen-, sulphur- or nitrogen-containing heterocyclic group which may optionally be substituted at the nitrogen atom by a C₁-C₃ alkyl group, and
R³ denotes a hydrogen or halogen atom,
from a 5-aryl-1,3-dihydrothieno[2,3-*e*]-1,4-diazepine-2-one of formula II 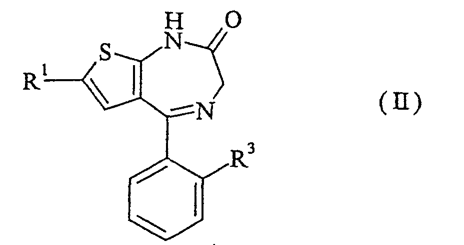 wherein R¹ and R³ are as herein defined, **characterised in that** the compound of formula II,
(a) is treated with a chlorinating agent,
(b) the 5-aryl-2-chlorothieno[2,3-*e*]-1,4-diazepine of formula III obtained, 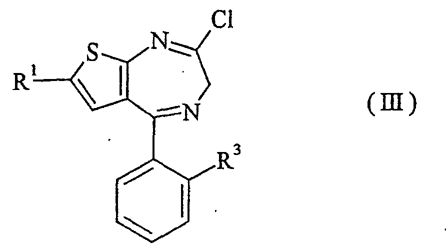 wherein R¹ and R³ are as herein defined, is reacted with an acylhydrazine of formula IV
R²-CO-NH-NH₂ (IV)
wherein R² is as hereinbefore defined, and
(c) the product thus obtained is treated with a base.

2. Process according to claim 1 for preparing a compound of formula I, wherein R¹ denotes a bromine atom, R² denotes a methyl group and R³ denotes a chlorine atom.

3. Process according to claim 1 or 2, **characterised in that** steps (a) to (c) are carried out in a one-pot process.

4. Process according to one of claims 1 to 3, **characterised in that** in step (a) the compound of formula (II) is reacted with phosphorus pentachloride in the presence of an inert diluent and a tertiary amine.

5. Process according to one of claims 1 to 4, **characterised in that** in step (b) the compound of formula (III) is reacted with acetic acid hydrazide at a temperature below 100 °C in the presence of an inert diluent.

6. Process according to one of claims 1 to 5, **characterised in that** in step (c) the compound obtained in step (b) is treated with aqueous sodium hydroxide solution at a temperature from 0 °C to 50 °C.

7. Process for preparing a compound of formula I, wherein R¹ denotes bromine, in which a compound of formula V, 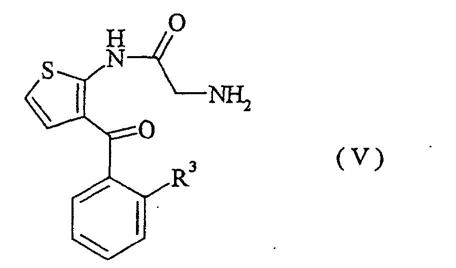 wherein R³ has the meaning given in claim 1 or 2,
is subjected to the following reactions one after another:
(d) cyclisation under water-cleaving conditions;
(e) reaction of the product obtained in step d) with a brominating agent;
(f) converting the compound of formula II thus obtained, wherein R¹ denotes bromine, into the compound of formula I according to one of claims 1 to 6,
**characterised in that** steps (d) and (e) are carried out in a one-pot process.

8. Process according to claim 7, **characterised in that** the compound of formula V is heated in step (d) with an acid in a high-boiling solvent.

9. Process according to claim 7 or 8, **characterised in that** the compound obtained in step (d) is reacted with bromine in the presence of a tertiary amine.

10. 5-Aryl-2-chlorothieno[2,3-*e*]-1,4-diazepine of formula III, 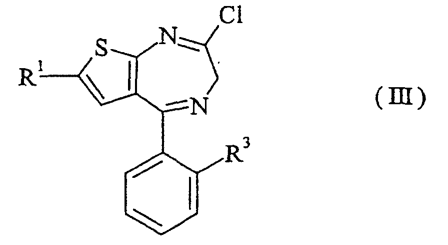 wherein R¹ and R³ have the meanings given in claim 1 or 2.

## Revendications

1. Procédé de préparation de 6-aryl-4H-s-triazolo[3,4-*c*]-thiéno[2,3-*e*]-1,4-diazépines de formule I 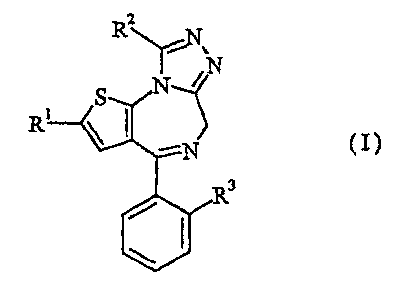 où
R¹ représente un atome d'hydrogène ou d'halogène ou un groupement C₁-C₆-alkyle ;
R² représente un atome d'hydrogène ou d'halogène ou un groupement C₁-C₆-alkyle, C₁-C₆-hydroxyalkyle, C₃-C₆-cycloalkyle ou un groupe hétérocyclique à 5 ou 6 chaînons contenant de l'oxygène, du soufre ou de l'azote, qui peut éventuellement être substitué sur l'atome d'azote par un groupement C₁-C₃-alkyle, et
R³ représente un atome d'hydrogène ou d'halogène,
à partir d'une 5-aryl-1,3-dihydrothiéno[2,3-*e*]-1,4-diazépin-2-one de formule II 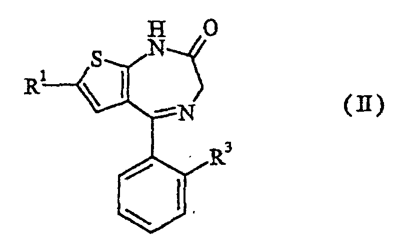 où R¹ et R³ présentent la signification indiquée, **caractérisé en ce que** l'on traite le composé de formule II
(a) avec un agent de chloration,
(b) on fait réagir la 5-aryl-2-chlorothiéno[2,3-*e*]-1,4-diazépine obtenue de formule III 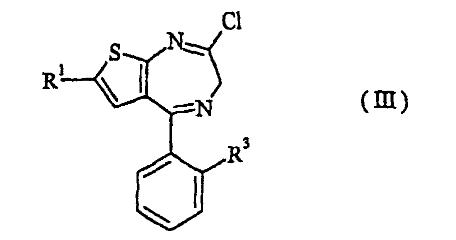 où R¹ et R³ présentent la signification indiquée, avec une acylhydrazine de formule IV
R²-CO-NH-NH₂ (IV)
où R² présente la signification indiquée, et
(c) on traite avec une base le produit ainsi obtenu.

2. Procédé selon la revendication 1 pour la préparation d'un composé de formule I où R¹ représente un atome de brome, R² représente un groupe méthyle et R³ représente un atome de chlore.

3. Procédé selon la revendication 1 ou 2 **caractérisé en ce que** l'on réalise les étapes (a) à (c) dans un procédé à récipient unique.

4. Procédé selon l'une des revendications 1 à 3 **caractérisé en ce que**, dans l'étape (a), on fait réagir le composé de formule (II) avec le pentachlorure de phosphore en présence d'un diluant inerte et d'une amine tertiaire.

5. Procédé selon l'une des revendications 1 à 4 **caractérisé en ce que**, dans l'étape (b), on fait réagir le composé de formule (III) à une température inférieure à 100°C avec l'acétohydrazide en présence d'un diluant inerte.

6. Procédé selon l'une des revendications 1 à 5 **caractérisé en ce que**, dans l'étape (c), on traite le composé obtenu dans l'étape (b) à une température de 0°C à 50°C avec de la lessive de soude aqueuse.

7. Procédé de préparation d'un composé de formule I où R¹ représente le brome où l'on soumet un composé de formule V 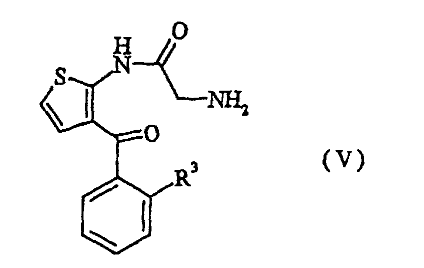 où R³ possède la signification indiquée dans la revendication 1 ou 2, successivement aux réactions suivantes :
(d) cyclisation dans des conditions éliminant de l'eau;
(e) réaction du produit obtenu dans l'étape d) avec un agent de bromation ;
(f) conversion du composé ainsi obtenu de formule II où R¹ représente le brome en le composé de formule I selon l'une des revendications 1 à 6,
**caractérisé en ce que** l'on réalise les étapes (d) et (e) dans un procédé à récipient unique.

8. Procédé selon la revendication 7 **caractérisé en ce que** l'on chauffe le composé de formule V dans l'étape (d) avec un acide dans un solvant à haut point d'ébullition.

9. Procédé selon la revendication 7 ou 8 **caractérisé en ce que** l'on fait réagir avec du brome en présence d'une amine tertiaire le composé obtenu dans l'étape (d).

10. 5-aryl-2-chlorothiéno[2,3-*e*]-1,4-diazépine de formule III 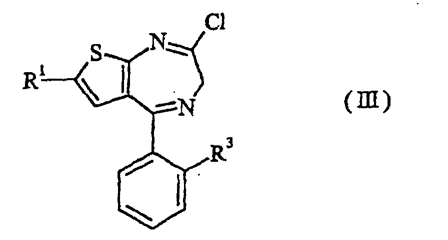 où R¹ et R³ présentent les significations indiquées dans la revendication 1 ou 2.
